# EUROPEAN PATENT SPECIFICATION

(11) **EP 1 535 619 B1**
(45) Date of publication and mention of the grant of the patent: **19.03.2008**
(21) Application number: 03026959.1
(22) Date of filing: 25.11.2003
(51) Int. Cl.: A61K 31/575, A61K 36/076, A61P 37/04

(54) **Poria extract devoid of secolanostane derivatives comprising lanostane derivatives for immunity enhancement**
Poria-Extrakt frei von Secolanostanderivaten, enthaltend Lanostanderivate zur Immunsteigerung
Extrait de Poria dépourvu de dérivés sécolanostane, comprenant des dérivés lanostane pour augmenter l'immunité

(43) Date of publication of application: 01.06.2005
(73) Proprietor: Sinphar Pharmaceutical Co., Ltd., Taipei (TW)
(72) Inventor: Lin, Hang-Ching, Taipei Taiwan (CN); Tseng, Jerming, Taipei Taiwan (CN); Ding, Hsiou Yu, Tainan Taiwan (CN); Chang, Wen-Liang, Taipei Taiwan (CN); Chao, Chien-Lian, Taoyuan Taiwan (CN); Huang, Hsin-Wen, Taipei Taiwan (CN)
(74) Representative: Grünecker, Kinkeldey, Stockmair & Schwanhäusser Anwaltssozietät

(56) References cited:
- CN-B- 1 008 183
- JP-A- 8 119 864
- JP-A- 9 025 232
- JP-A- 2001 206 830
- DATABASE WPI Section Ch, Week 200112 Derwent Publications Ltd., London, GB; Class B04, AN 2001-103728 XP002279243 & CN 1 146 915 A (WANG L), 9 April 1997 (1997-04-09)
- LIOU CHIAN-JIUN ET AL: "A Chinese herbal medicine, fu-ling, regulates interleukin-10 production by murine spleen cells." THE AMERICAN JOURNAL OF CHINESE MEDICINE. UNITED STATES 2002, vol. 30, no. 4, 2002, pages 551-560, XP009030597 ISSN: 0192-415X
- YU SHU-JIUAN ET AL: "Fu-Ling, a Chinese herbal drug, modulates cytokine secretion by human peripheral blood monocytes" INTERNATIONAL JOURNAL OF IMMUNOPHARMACOLOGY, vol. 18, no. 1, 1996, pages 37-44, XP002279242 ISSN: 0192-0561
- TSENG JERMING ET AL: "Si-Jun-Zi-Tang regulate granulocyte macrophage colony-stimulating factor secretion by human peripheral blood mononuclear cells" AMERICAN JOURNAL OF CHINESE MEDICINE, vol. 24, no. 1, 1996, pages 45-52, XP009030596 ISSN: 0192-415X

## Description

The present invention relates to a *Poria* extract in accordance with the appended claims containing lanostane as a potent component thereof for enhancing immunity. The present invention relates in particular to an extract of *Poria cocos* (Schw) Wolf for the purpose of immunity enhancement.

The *Poria* extract has tonic effect, as well as a smoothing effect on stomach disorder. According to the Chinese medicine, the *Poria* extract is classified as a tranquilizer and a uretic agent. In addition, the *Poria* extract is used as one of essential ingredients of the Chinese medicine prescription for vital activity. On the basis of researches and experiments, which have been conducted in recent years on the pharmacological effect of the *Poria* extract, it has been concluded that the *Poria* extract has a favorable effect on tumor prevention, and that the *Poria* extract is beneficial to immunity enhancement and gastrointestinal system of a person suffering from a chronic disease.

As exemplification, the Japan Patent Publication Numbers 55-111791 and 57-038794 disclose an extract which is obtained from the cultivated mycelia of *Poria cocos* (Schw) Wolf and is effective in tumor prevention. The Japan Patent Publication No. 55-111422 discloses an extract which is directly obtained from *Poria cocos* (Schw) Wolf for use in tumor prevention. The Japan Patent Publication No. 8-119864 discloses an extract which is obtained by extracting *Poria cocos* (Schw) Wolf with methanol. By separation, triterpene compounds such as lanostanes and secolanostanes are obtained from the extract and are used as anti -emetic agents. The Japan Patent Publication No. 9-025232 discloses triterpene compounds, which is obtained by extracting *Poria cocos* (Schw) Wolf with methanol. The compounds are useful as a tumor promotion-inhibiting agent. The Japan Patent Publication No. 9-176184 discloses an extract of *Poria cocos* (Schw) Wolf, which is then refined to produce triterpene compounds for use as an agent for inhibiting inflammation and tumor promotion.

The China Patent Publication No. 1008183 discloses a method of making a *Poria* extract containing triterpene compounds. The method involves extracting the *Poria* powder with an acidic alcohol, neutralizing the extract with a basic solution, concentrating the neutralized solution, adjusting the pH thereof to about 10 and filtering the solution, acidifying the filtrate to form a precipitate, washing the precipitate after filtration, and drying the washed precipitate. The *Poria* extract so obtained is found to have a tumor-preventive effect and an immunity activation effect.

CN 1146915 discloses an anticancer composition, which contains coix seed polyose, yam polyose, pachyman, coixenolide, pachymic acid and polystictin and can serve to improve immunity and increase leucocytes for patient after chemotherapy.

JP2001206830 discloses a composition for the oral cavity, which has a highly inhibitory effect on dental plaque formation. This composition uses both a water-soluble or sparingly water-soluble β-D-glucan and one or more kinds selected from compounds belonging to monoterpene, sesquiterpene, diterpene and triterpene.

The object of the present invention is to provide a new *Poria* extract for enhancing immunity of a mammal.

The object is achieved by a *Poria* extract capable of enhancing immunity of a mammal comprising 5-60% of a lanostane having the following chemical formula (I) by weight of the extract, and being substantially devoid of secolanostane: 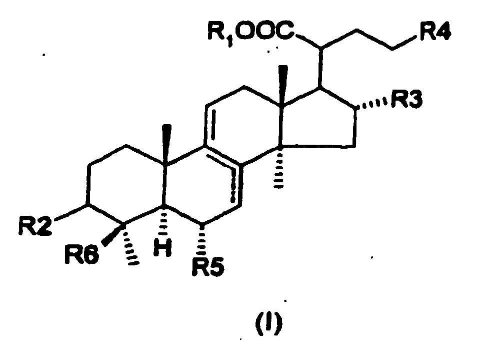 wherein R₁ is either H or CH₃; R₂ is OCOCH₃, =O or OH; R₃ is H or OH; R₄ is -C(=CH₂)-C(CH₃)₂Rₐ, wherein Rₐ is H or OH, or -CH=C(CH₃)-R_{b}, wherein R_{b} is CH₃ or CH₂OH; R₅ is H or OH; and R₆ is CH₃ or CH₂OH.

The present invention makes use of the immunity experiments to verify the pharmacological properties of the potent component of the *Poria* extract. The potent component is a low polarity portion (PCM), which contains major compounds of K1, K2, K3, K4, and trace of K4a, K4b, K5, K6a, K6b, which are all lanostane compounds. The compounds of K1; K2, K3, and K4 have an immunity enhancement effect.

The Poria extract of the present invention is prepared by a method, which makes use of the conventional extraction process, by means of which a crude extract is obtained. A chromatographic separation is used to separate constituents of the crude extract, which include a lanostane fraction and a secolanostane fraction. The lanostane fraction is relatively smaller in polarity than the secolanostane fraction. The lanostane fraction is obtained by using an eluent made of dichloromethane : methanol = 96:4, whereas the secolanostane fraction is obtained by using an eluent made of dichloromethane : methanol = 90:10 or 0:100. The position of the lanostane fraction is identified by the thin layer chromatography, which has a chromatographic value (Rf) being . 0.1 when a developing solution of dichloromethane : methanol (96:4) is used. The chromatographic value of the secolanostane fraction is smaller than 0.1. By a silica gel column chromatography, the lanostane fraction is separated into several lanostane compounds with an eluent made of dichloromethane : methanol alcohol = 97:3 to 95:5.

According to the method, 2.6 grams of PCM are obtained from one kilogram of *Poria cocos* (Schw) Wolf. According to the method of the China Patent Publication No.1008183, three grams of a crude extract are obtained from one kilogram of *Poria cocos* (Schw) Wolf. When this crude extract is purified by the method of the present invention, only one gram of PCM is obtained.

The features of the present invention will be more readily understood upon a thoughtful deliberation of the following detailed description of the embodiments of the present invention with reference to the accompanying drawings.

### BRIEF DESCRIPTION OF THE DRAWINGS

FIG. 1 shows a flow diagram of a method for making lanostane compounds from *Poria cocos* (Schw) Wolf.
FIG. 2 shows a flow diagram of making a *Poria* extract in accordance with a method disclosed by the China Patent Publication No. 1008183, and a low polarity portion of the *Poria* extract according to the present invention.

### DETAILED DESCRIPTION OF THE INVENTION

The inventors of the present invention repeated the example disclosed in the China Patent Publication No. 1008183 by using *Poria cocos* (Schw) Wolf, which was grown in China. By an acid extraction and alkaline/acidic treatments, 3 grams of a crude *Poria* extract were obtained from one kilogram of *Poria cocos* (Schw) Wolf. This is in agreement with the range of 2.5 g ± 0.5 g disclosed in the afore-mentioned China Patent Publication. As a result of further separation, 400 mg of purified lanostane compounds were obtained. In another words, the extract obtained according to the method disclosed in the afore-mentioned China Patent publication contains about 13% of lanostane fraction, with 87% of the extract being the secolanostane fraction and other unidentified constituents.

As a result of further experiments carried out by the inventors of the present invention, the lanostane fraction has no toxic effect on vat spleen cells and is pharmacologically effective. The secolanostane fraction has a toxic effect on rat spleen cells.

The present invention discloses a Poria extract capable of enhancing immunity of mammal, such as Homo sapiens. The Poria extract comprises 5-60% of a lanostane having the following chemical formula (I) by weight of the extract, and being substantially devoid of secolanostane: 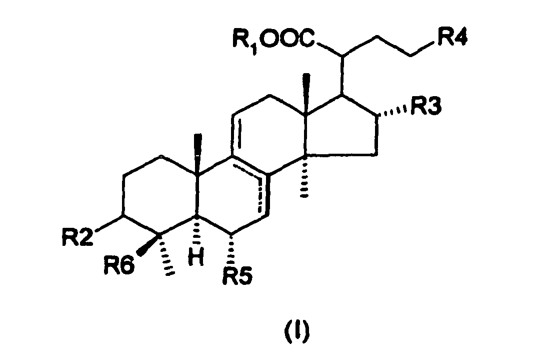 wherein R₁ is either H or CH₃; R₂ is OCOCH₃, =O or OH; R₃ is H or OH; R₄ is -C(=CH₂)-C(CH₃)₂Rₐ, wherein Rₐ is H or OH, or -CH=C(CH₃)-R_{b}, wherein R_{b} is CH₃ or CH₂OH; R₅ is H or OH; and R₆ is CH₃ or CH₂OH.

The Poria extract contains 5-60%, preferably 10-20%, of the lanostane (I) by weight of the extract.

A method for the preparation of the *Poria* extract is described. The method includes a first step in which the metabolites, the fermentation products, and the sclerotium of *Poria cocos* (Schw) Wolf are extract by a solvent, such as water, methanol, ethanol, or a mixture thereof, thereby resulting in production of a liquid extract, which is then concentrated to form a concentrated substance. The concentrated substance is introduced into a silica gel column, and is eluted with an eluent having a low polarity. As a result, an eluate is produced and collected. The eluate is concentrated to form a concentrated eluate, which has a chromatographic value (Rf) . 0.1 in accordance with a thin layer chromatography, which is developed by a mixed solvent of dichloromethane : methanol = 96:4 and is detected by an ultraviolet lamp and iodine vapor.

It is suggested that the extraction is carried out by using 95% ethanol.

Preferably, the concentrated substance is further extracted with a two-phase solvent containing methanol and n-hexane in a volumetric ratio of 1:1. The methanol layer is separated and is concentrated to form a concentrate, which is used as a feed to the silica gel column.

It is recommended that the low polarity eluent is a mixed solvent containing dichloromethane and methanol in a volumetric ratio of 96.5:3.5.

Preferably, the lanostane (I) has the following structures:

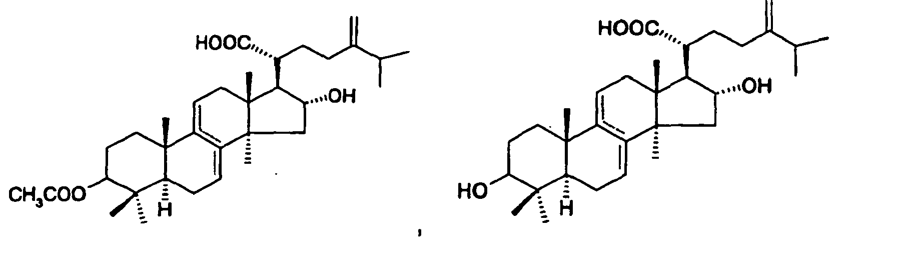

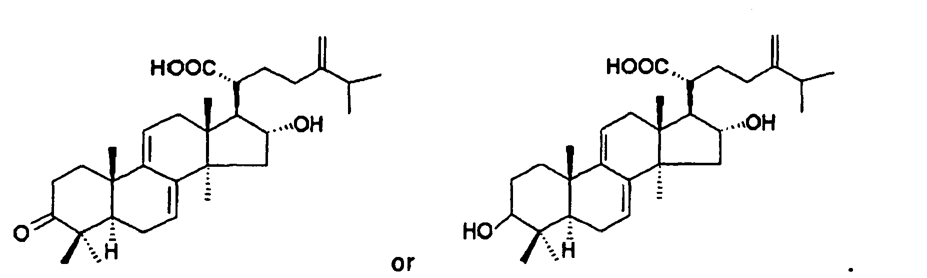

The present invention also discloses a use of the *Poria* extract of the present invention in the manufacture of a medicament for enhancing immunity of mammal.

### Example 1

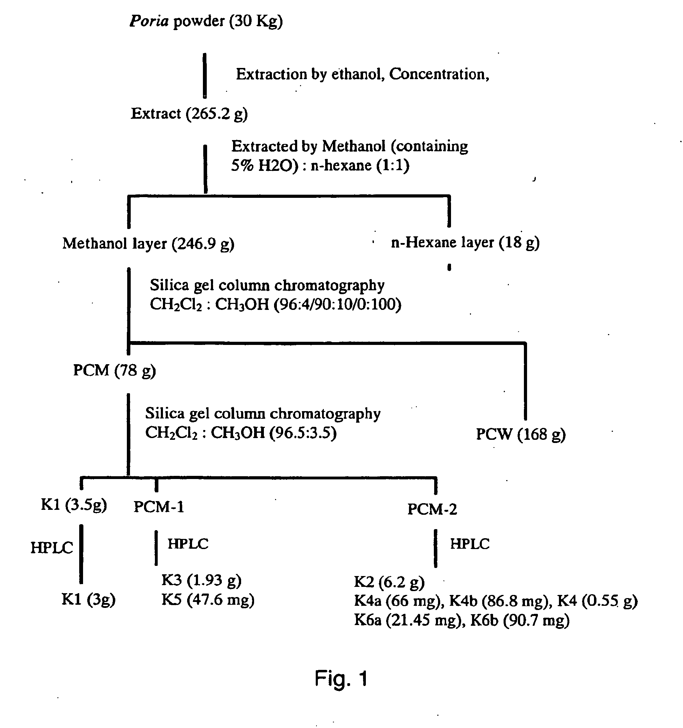

As illustrated in FIG. 1, a *Poria* powder was made of 30 kilograms of the China-grown *Poria cocos* (Schw) Wolf. The *Poria* powder was extracted with 120 L 95% alcohol for 24 hours. The mixture was filtered to obtain a filtrate. The residue was extracted and filtered for another three cycles. The filtrates were combined and concentrated to bring about a dried extract in amount of 265.2 grams. The dry extract was undergone a distribution extraction with a two-phase extraction agent (n-hexane : 95% methanol = 1:1), and the methanol layer was removed therefrom, which is then concentrated to obtain a dry solid in an amount of 246.9 grams. A separation of the dry solid was carried out by means of a silica gel column, which was filled with silica gel 10-40 times of the weight of the dry solid. The silica gel having a diameter of 70-230 mesh was made by Merck corporation with a code of Silica Gel 60. The column was eluted by the following eluates in sequence: a mixed solvent of dichloromethane : methanol = 96:4; a mixed solvent of dichloromethane : methanol = 90:10, and pure methanol. The eluates were tested by the thin layer chromatography (TLC), wherein an ultraviolet lamp and iodine vapor were used for detecting, and a mixed solvent of dichloromethane : methane = 96:4 was used as a developing liquid. The eluates having similar constituents in the TLC were combined.

The elution carried out with the mixed solvent of dichloromethane : methanol = 96:4 resulted in a PCM portion in amount of 78 grams. The PCM shows 6 trace points in the thin layer chromatography. The resulting eluates from the elutions carried out with the eluents of dichloromethane : methanol = 90:10 and pure methanol were combined to obtain a PCW portion in amount of 168 grams.

The PCM portion was further separated by means of an eluent of dichloromethane : methanol = 96.5:3.5 and the silica gel column. With the thin layer chromatography, the eluates were collected as three fractions, which were respectively K1 (Rf=0.64), PCM-1 (containing trace K1, K3 (Rf=0.55), K5 (Rf=0.49)), and PCM-2 (containing K2 (Rf=0.30), K4 (Rf=0.24), K6 (Rf=0.19). The K1 fraction (3.5g) was further subjected to a high performance liquid chromatography (HPLC) using a carbon-18 column and a mobile phase of methanol-water (90:10)). 3.0 grams of K1 component was obtained.

With the same HPLC and a mobile phase of methanol-water (87:13), the PCM-1 fraction was separated into K3, K5, and trace K1 components. The K3 component was further purified to yield K3 (1.93g) by means of the same HPLC and a mobile phase of methanol-water (84:16). The K5 component was further purified with the same HPLC and two mobile phases of methanol-water (93:7) and (91:9) in sequence to yield K5 (47.6mg).

With the same HPLC and a mobile phase of methanol-water (87:13), the PCM-2 fraction were separated into K6 trace component (K6a + K6b), K4 trace component (K4a + K4b), K2 component, and K4 component. By making use of the same HPLC and a mobile phase of methanol-water (84:16), the K2 component and the K4 component were further purified to yield K2 (6.2g) and K4 (0.55g).

With the same HPLC and a mobile phase of CH₃CN-water (68:32), the K6 trace component was purified to yield K6a (21.4mg) and K6b (90.7mg). The K4 trace component was subjected to the same HPLC and a mobile phase of methanol-water (76:24) to yield K4a (66.0mg) and K4b (86.8mg).

The afore-mentioned K1-K6 compounds have analytical data as follows:
K1: mixture, EI-MS: major component, 528[M]⁺; trace component, 526[M]⁺ K1 (major component): ¹³C-NMR (δ c):35.4 (c-1), 24.5 (c-2), 80.6 (c-3), 38.0 (c-4), 50.7 (c-5), 18.4 (c-6), 26.8 (c-7), 135.0 (c-8), 134.4 (c-9), 37.2 (c-10), 20.9 (c-11), 29.7 (c-12), 48.8 (c-13), 46.3 (c-14), 43.6 (c-15), 26.6 (c-16), 57.3 (c-17), 17.8 (c-18), 19.2 (c-19), 48.6 (c-20); 178.6 (c-21), 31.6 (c-22), 33.2 (c-23), 156.1 (c-24), 34.1 (c-25), 22.0 (c-26), 21.9 (c-27), 28.0 (c-28), 16.8 (c-29), 25.4 (c-30), 107.0 (c-31), 21.1 (CH₃COO-), 170.5 (CH₃COO-)
K1 (trace component): ¹³C-NMR (δ c): 35.6 (c-1), 24.5 (c-2), 80.6 (c-3), 37.8 (c-4), 49.7 (c-5), 23.1 (c-6), 120.6 (c-7), 142.8 (c-8), 145.8 (c-9), 37.6 (c-10), 117.0 (c-11), 36.3 (c-12), 49.4 (c-13), 45.1 (c-14), 44.4 (c-15), 76.4 (c-16), 57.6 (c-17), 17.6 (c-18), 22.8 (c-19), 48.4 (c-20), 178.5 (c-21), 31.4 (c-22), 33.2 (c-25), 156.0 (c-24), 34.1 (c-25), 22.0 (c-26), 21.9 (c-27), 28.2 (c-28), 17.1 (c-29), 26.5 (c-30), 107.0 (c-31), 21.1 (CH₃COO-), 170.4 (CH₃COO-)
K2: mixture, EI-MS: major component, 486[M]⁺; trace component, 484[M]⁺ K2 (major component): ¹³C-NMR (δ c): 36.6 (c-1), 29.1 (c-2), 78.5 (c-3), 40.0 (c-4), 51.4 (c-5), 19.2 (c-6), 27.4 (c-7), 135.4 (c-8), 135.3 (c-9), 37.9 (c-10), 21.4 (c-11), 30.2 (c-12), 49.3 (c-13), 46.7 (c-14), 44.2 (c-15), 77.1 (c-16), 57.8 (c-17), 18.2 (c-18), 19.8 (c-19), 49.2 (c-20), 179.4 (c-21), 32.1 (c-22), 33.7 (c-23), 156.5 (c-24), 34.6 (c-25), 22.5 (c-26), 22.4 (c-27), 29.1 (c-28), 16.8 (c-29), 25.9 (c-30), 107.5 (c-31)
K2 (trace component): ¹³C-NMR (δ c): 36.7 (c-1), 29.1 (c-2), 78.5 (c-3), 40.0 (c-4), 49.8 (c-5), 24.3 (c-6), 121.2 (c-7), 143.3 (c-8), 145.2 (c-9), 38.0 (c-10), 118.1 (c-11), 37.2 (c-12), 45.5 (c-13), 49.1 (c-14), 44.8 (c-15), 76.8 (c-16), 58.0 (c-17), 18.1 (c-18), 22.9 (c-19), 48.0 (c-20), 179.4 (c-21), 31.9 (c-22), 33.7 (c-23), 156.5 (c-24), 34.6 (c-25), 22.9 (c-26), 22.4 (c-27), 29.1 (c-28), 16.8 (c-29), 26.8 (c-30), 107.5 (c-31)
K3: mp: 278-280°C
   [α]_{D}²⁴+3° (c 0.6, Pyridine)
   EI-MS m/z: 482[M]⁺, ¹³C-NMR (δ c): 37.5 (c-1), 35.7 (c-2), 216.7 (c-3), 48.3 (c-4), 51.8 (c-5), 24.6 (c-6), 121.4 (c-7), 143.5 (c-8), 145.4 (c-9), 38.2 (c-10), 118.3 (c-11), 36.9 (c-12), 45.7 (c-13), 50.0 (c-14), 44.9 (c-15), 77.2 (c-16), 58.1 (c-17), 18.3 (c-18), 22.7 (c-19), 49.2 (c-20), 179.6 (c-21), 32.0 (c-22), 33.8 (c-23), 156.7 (c-24), 34.8 (c-25), 22.7 (c-26), 22.6 (c-27), 26.3 (c-28), 23.1 (c-29), 27.1 (c-30), 107.8 (c-31)
K4: mp:>300°C
   [α]_{D}²⁴+18° (*c* 0.5, Pyridine)
   EI-MS m/z: 484[M]⁺, ¹³C-NMR (δc): 31.4 (c-1), 27.4 (c-2), 76.0 (c-3), 38.6 (c-4), 44.5 (c-5), 24.2 (c-6), 122.0 (c-7), 143.5 (c-8), 147.4 (c-9), 38.6 (c-10), 116.9 (c-11), 37.0 (c-12), 45.9 (c-13), 50.2 (c-14), 45.1 (c-15), 77.3 (c-16), 58.2 (c-17), 18.4 (c-18), 23.7 (c-19), 49.3 (c-20), 179.8 (c-21).32.1 (c-22), 33.9 (c-23), 156.7 (c-24), 34.9 (c-25), 22.8 (c-26), 22.6 (c-27), 29.9 (c-28), 23.9 (c-29), 27.3 (c-30), 107.9 (c-31)
K4a: mp: 284-287°C
   [α]_{D}²⁴+44° (c 0.5, Pyridine)
   EI-MS m/z: 498[M]⁺, ¹³C-NMR (δ c): 35.9 (c-1), 37.1 (c-2), 217.3 (c-3), 53.5 (c-4), 43.9 (c-5), 24.5 (c-6), 121.5 (c-7), 143.7 (c-8), 144.9 (c-9), 37.9 (c-10), 119.0 (c-11), 36.9 (c-12), 45.9 (c-13), 50.0 (c-14), 45.0 (c-15), 77.3 (c-16), 58.2 (c-17), 18.5 (c-18), 23.3 (c-19), 49.4 (c-20), 179.9 (c-21), 32.2 (c-22), 34.1 (c-23), 157.0 (c-24), 34.9 (c-25), 22.8 (c-26), 22.7 (c-27), 19.4 (c-28), 67.5 (c-29), 26.9 (c-30), 107.8 (c-31)
K4b: mp: 230-232°C
   [α]_{D}²⁴+38° (c 0.5, Pyridine)
   EI-MS m/z: 542[M]⁺, ¹³C-NMR (δ c):
   36.6 (c-1), 25.0 (c-2), 82.1 (c-3), 39.4 (c-4), 56.7 (c-5), 68.9 (c-6), 129.2 (c-7), 142.1 (c-8), 145.8 (c-9), 39.2 (c-10), 117.9 (c-11), 36.9 (c-12), 45.8 (c-13), 49.9 (c-14), 44.9 (c-15), 77.3 (c-16), 58.1 (c-17), 18.4 (c-18), 24.8 (c-19), 49.3 (c-20), 179.9 (c-21), 32.1 (c-22), 33.9 (c-23).156.7 (c-24), 34.9 (c-25), 22.8 (c-26), 22.7 (c-27), 31.9 (c-28), 18.1 (c-29), 27.1 (c-30), 107.9 (c-31), 22.0 (CH₃COO-), 172.0 (CH₃COO-)
K5: mp: 274-275°C
   [α]_{D}²⁴+10° (c 0.5, Pyridine)
   EI-MS m/z: 454[M]⁺ , ¹³C-NMR (δ c):
   36.8 (c-1), 29.6 (c-2), 79.0 (c-3), 38.6 (c-4), 50.6 (c-5), 24.3 (c-6), 122.1 (c-7), 143.6 (c-8), 147.3 (c-9), 37.2 (c-10), 117.5 (c-11), 34.1 (c-12), 45.0 (c-13), 51.3 (c-14), 30.8 (c-15), 28.1 (c-16), 48.9 (c-17), 17.4 (c-18), 23.7 (c-19), 49.9 (c-20), 179.9 (c-21), 32.4 (c-22), 27.5 (c-23), 124.3 (c-24), 132.7 (c-25), 26.6 (c-26), 18.6 (c-27), 29.2 (c-28), 17.1 (c-29), 26.7 (c-30)
K6a: mp: 248-250°C
   [α]_{D}²⁴+63° (c 0.4, Pyridine)
   EI-MS m/z: 498[M]⁺, ¹³C-NMR (δ c): 37.1 (c-1), 35.0 (c-2), 219.0 (c-3), 48.4 (c-4), 57.0 (c-5), 68.0 (c-6), 128.6 (c-7), 141.8 (c-8), 144.2 (c-9), 38.3 (c-10), 120.2 (c-11), 36.6 (c-12), 45.8 (c-13), 49.7 (c-14), 44.8 (c-15), 77.2 (c-16), 58.1 (c-17), 18.4 (c-18), 22.7 (c-19), 49.4 (c-20), 179.6 (c-21), 32.1 (c-22), 33.9 (c-23), 156.8 (c-24), 34.9 (c-25), 22.8 (c-26), 22.7 (c-27), 31.5 (c-28), 24.5 (c-269), 26.6 (c-30), 107.9 (c-31)
K6b: mp: 267-270°C
   [α]_{D}²⁴+68° (c 0.3, Pyridine)
   EI-MS m/z: 51 6[M]⁺, ¹³C-NMR (δ c): 34.4 (c-1), 29.4 (c-2), 74.1 (c-3), 42.6 (c-4), 87.7 (c-5), 133.1 (c-6), 134.7 (c-7), 79.6 (c-8), 145.8 (c-9), 42.1 (c-10), 120.7 (c-11), 36.8 (c-12), 49.1 (c-13), 48.7 (c-14), 42.7 (c-15), 76.8 (c-16), 57.6 (c-17), 19.0 (c-18), 29.3 (c-19), 49.1 (c-20), 179.6 (c-21), 32.2 (c-22), 33.9 (c-23), 156.8 (c-24), 34.9 (c-25), 22.9 (c-26), 22.7 (c-27), 25.1 (c-28), 20.3 (c-29), 20.8 (c-30), 107.9 (c-31)
The structures of K1 to K6 compounds are listed as follows: 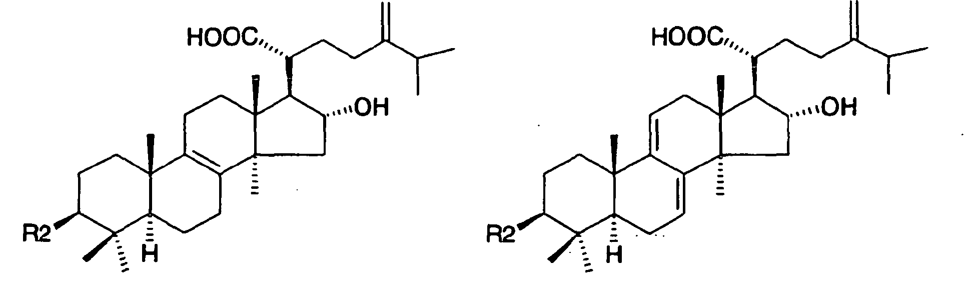 K1: R₂ = OCOCH₃, major component K1: R₂ = OCOCH₃, trace component
   K2:R₂ = OH, major component K2: R₂ = OH, trace component 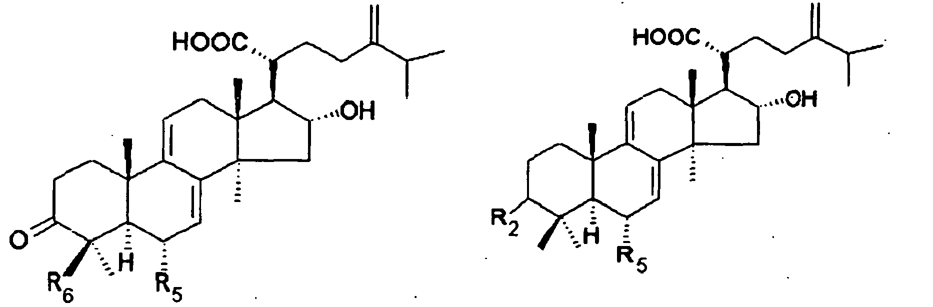 K3:R₆=CH₃ A₅=H K4: R₂ = α-OH R₅ = H
   K4a :R₆ =CH₂OH R₅= H K4b: R₂ = β -OCOCH₃ R₅ = OH
   K6a : R₆ = CH₃ R₅= OH

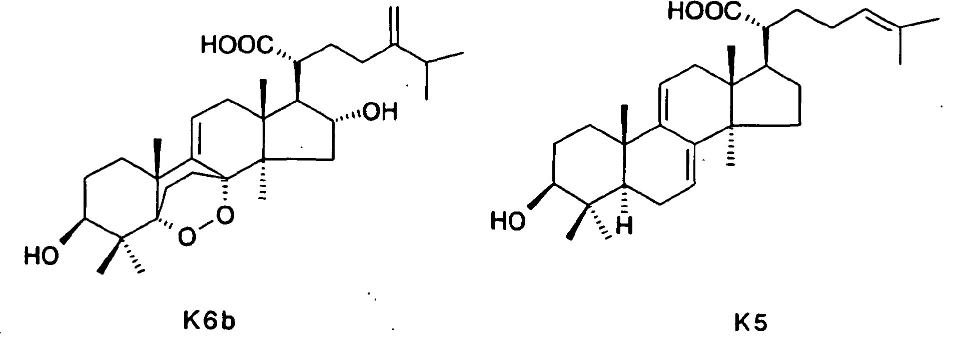

### Example 2

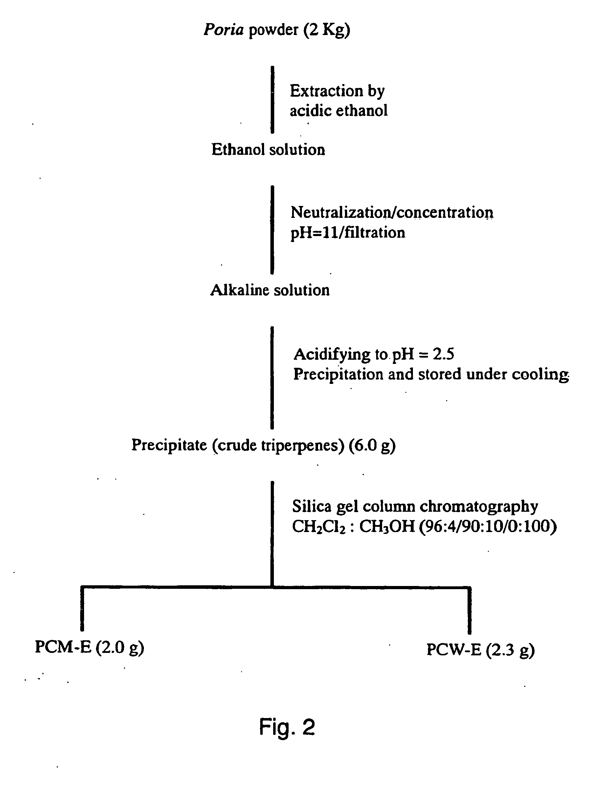

A powder was made of two kilograms of the China-grown *Poria cocos* (Schw) Wolf. By using the method disclosed in the China Patent Publication No. 1008183, a crude extract in an amount of 6.0g was obtained, as shown in FIG. 2. Following the procedures described in Example 1, the crude extract was subjected to a silica gel column chromatography and eluted with a mixed solvent of dichloromethane : methanol = 96:4 to obtain a PCM-E portion (2.0g), and eluted with dichloromethane : methanol = 90:10 and pure methanol in sequence to obtain a PCW-E portion (2.3g).

The PCM-E and the PCW-E prepared in Example 2 were orally administered to animals in a dose of 40mg/kg per day for the purpose of testing the effect of these two portions on growth of spleen cells (immunity cells) of the animals. The immune system of the animals will be enhanced, if the growth of spleen cells thereof are stimulated; and will be adversely affected, if the growth of spleen cells are inhibited, i.e. the spleen cells are killed due to toxicity.

The spleen cells were cultivated in vitro for five days before they were compared for cellular growth by means of MTT assay, which was determined by an immunological research method described hereinafter. The results are listed in the Table 1. The cellular growth of the spleen of the mice was apparently promoted on the third day and the fourth day in the wake of oral administration of PCM-E. Based on the statistic view point, there is no difference in terms of cellular growth among the control group and the experimental group. However, the number of the alive spleen cells of the mice was apparently lower than that of the control group on the third day and the fourth day in the wake of oral administration of PCW-E. In another words, the cellular growth of the spleen of the experimental group is apparently weakened as compared to the control group. This means that PCW-E is cytotoxic.

The implication is that the lanostane-containing low polarity portion, PCM, has no inhibitive effect on cellular growth of the spleen. In addition, the PCM portion has a promotive effect on cellular growth of the spleen. The secolanostane-containing high polarity portion (Rf<0.1) has an inhibitive effect on cellular growth of the spleen. The *Poria* extract of the present invention is devoid of the secolanostane fraction (PCW -E). On the contrary, the *Poria* extract obtained by the prior art methods contains the PCW-E capable of inhibiting cellular growth of the spleen.

**TABLE 1 Effects of PCM-E and PCW-E on cellular growth of spleen**

| component | dose | In vitro growth (day) | | |
|---|---|---|---|---|
| | (mg/kg/day) | 3rd day | 4th day | 5th day |
| Control group | | 0.608±0.042^{a} | 0.777±0.141 | 0.515±0.055 |
| PCM-E | 40 | 0.890±0.195 | 0.857±0.137 | 0.449±0.083 |
| PCW-E | 40 | 0.245±0.072* | 0.451±0.020** | 0.344±0.132 |

| | | | | |
|---|---|---|---|---|
| a: data are mean±S.E.M. of five experiments * is indicative of P< 0.05; * * is indicative of P<0.01 | | | | |

On the basis of the above two examples and the comparative results, the following facts are established.

The potent component of the *Poria cocos* (Schw) Wolf is a lanostane-containing low polarity portion, which is capable of enhancing immunity of human body.

A silica gel thin layer chromatography and a silica gel column chromatography can be employed to separate the promotive components from the inhibitive components of *Poria cocos* (Schw) Wolf.

The *Poria* extract obtained by the aforementioned method is devoid of the inhibitive components, which are secolanostanes and high polar molecules contained in the high polarity portion, PCW-E.

The animal experiments of the present invention are carried out by an oral administration of PCM-E and PCW-E to animals. In another words, the administrations of PCM-E and PCW-E take place in vivo other than in vitro. Because in vitro study of pharmaceutical compound on spleen cells dose not reflect actual effects of the pharmaceutical compound on cellular growth of the animal spleen in light of absence of interactions between cellular metabolism and the pharmaceutical compound. It is therefore readily apparent that the present invention is reliable and meaningful.

The *Poria* extract and the purified compounds from the *Poria* extract by chromatography prepared according to the aforementioned method were tested by various immunity response experiments listed in the following. The PCM fraction of *Poria* extract and the lanostane compounds K1, K2 , K3 and K4 purified therefrom in Example 1 exhibit immunity enhancement activities in immune cells (T cells/B cells) in the following immunity response tests, and, as shown in Table 2 to Table 10, K1, K2 , K3 and K4 compounds are potent at dosage low than 2.5 or 5.0 mg/kg.

### Experiments

### Experimental animals

BALB/c male mice, 6-8 weeks old, were used throughout the experiment. The animals were purchased from National Laboratory Animal Center, Taipei, Taiwan. The mice were housed in the Individual Ventilation Cage System, which provided an environment of Specific Pyrogen-Free. The facilities had 12-h day/night rhythm, 24-26°C and 30-70% humidity in air. Mice were fed autoclaved water and rodent chow, and the bedding was sterilized by autoclave also. The feedstock purchased from animal center of National Taiwan University composed of crude proteins (>23.0%), crude fat (>4.5%), crude fiber (<6.0%), ash (<8.0%), added minerals (<3.0%) and water (<12%). After transport, the mice were allowed to rest for two weeks before the experiments were started.

### Animal treatment

For the drug treatment, the animals were divided into four groups and oral administration with 1 ml of PCM, ranging from 10, 40 to 80 mg/kg/day, for four days. For the studies on the purified PCM -K1, K2, K3 and K4, the dosage of oral administration had a range from 2.5, 5, 10 to 20 mg/kg/day, which were a quarter of the dosage used for PCM study. The control group was the mice feeding with an equal volume of saline (0.85% of NaCl). Mice were sacrificed at day five, and both the serum and spleen cells were collected. The sera were subsequently used to measure the concentration of IgG, IgM and IgA. The spleen cells were plated onto a 100 mm diameter culture plate and incubated at 37°C for three hours. The non-adherent cells containing T-lymphocytes, B-lymphocytes and NK cells were collected and properly diluted as described in each assay.

### Drug preparation

The dry powder of PCM, PCM-K1, K-2, K-3 and K4 were suspended in sterile water, followed by sonication to break down the large particle. The suspension of fine particles was used to feed the animals.

### Isolation of spleen lymphocytes

The mouse to be sacrificed was cervical dislocated and sterilized with 70% ethanol. The cardiac punctuation was performed first to withdraw the blood. After the haemagglutination, the blood sample was subjected to centrifugation and the serum was collect. For isolation of spleen cells, the peritoneum was opened to remove the spleen. The fresh spleen was transferred to a culture plate containing 10 ml of RPMI-1640 culture medium. The spleen was then ground over a fine mesh to release the spleen cells. The spleen cells suspending in the medium were then removed and transferred to a 50-ml conical centrifuge tube. The tube was subjected to centrifugati on at 1300 rpm for 10 min. The supernatant was discarded and the pellet was re-suspended in 1 ml of cold RBC lysing buffer containing EDTA-NH₄Cl. The cells were incubated at room temperature for 10 min, followed by washing the cells three times with culture medium by centrifugation. The spleen cells were plated onto a 100 mm diameter culture plate and incubated at 37°C for three hours. The non-adherent cells containing T-lymphocytes, B-lymphocytes and NK cells were collected and properly diluted as described in each assay.

### MTT assay

MTT assay is the method routinely used for estimating the viable cell number and viability of the cultured cells. The basic principle is that the only mitochondria in a viable cells contain biologically activated oxido-reductive enzymes. The enzymes interact with MTT reagents to convert the chemical into a relatively insoluble blue crystal. The crystal is then solublized in an acidic isopropanol and the absorbence at 570nm in each well was read using an ELISA reader (EL311, BioTek, VT). Briefly, the spleen cells suspended in a medium containing RPMI 1640 supplemented with 10% fetal bovine serum (FBS), 2 mM L-glutamine, antibiotics and 1 µg/ml concanavalin A (Con A) were cultured in the 96-well flat-bottom plate (5 x 10⁵cells/100 µl/well) for 5 days. MTT assay was performed at day 3, 4 and 5. MTT tetrazolium (3-(4-5-dimethylthiazol-2-yl)-2,5-diphenyltertrazolium bromide) (5 mg/ml in phosphate-buffered saline) (Sigma Chemical, St. Leuis, MO) was added to the spleen cells (20 µl MTT per 100µl cells), and plates were incubated at 37°C for 4 h. Acid-isopropanol (100 µl of 0.04 N HCL in isopropanol) was added to all wells and mixed completely to dissolve the dark blue crystals. After 20 minutes at room temperature to ensure that all crystals were dissolved, the plates were read on an EIA reader at 570 nm.

### ELISA to measure concentration of immunoglobulins

An ELISA was performed in our study to measure the immunoglobulin concentration. Briefly, the spleen cells (5x10⁵ cells/ml) were cultured in a medium containing RPMI 1640 supplemented with 10% fetal bovine serum (FBS), 2 mM L-glutamine, antibiotics and 5 µg/ml lipopolysaccharide (LPS; 1 µg/10⁵ cells) for 5 days. LPS is a polyclonal activator of B lymphocyte. Culture spleen cell supernatants were collected at day 3, 4 and 5. IgA, IgG and IgM concentrations were measured using a sandwich ELISA technique.

### 1. IgG assay:

A 96-well microtiter plate (Nunc-Immuno Plate, MaxiSorp, Nunc, Denmark) was precoated with 100 ng/well of capture antibodies at 4°C overnight. The capture antibody was a rabbit anti-mouse IgG + IgA + IgM antibody (Zymed Laboratory, CA). The plate was washed with PBS-0.05% Tween 20 solution and blocked with PBS-1% gelatin. After the blocking, the properly diluted samples (1/10⁵ diluted) and standard IgG ranged from 0.25 µg/ml to 0.039 µg/ml were added (100µl/well). The plate was then incubated at 37°C for 2 h. At the end of incubation, HRP-conjugated goat anti-mouse IgG (1:2,000 diluted; anti-whole IgG molecule; Zymed Laboratory, CA) was added (100 µl/well). After 1 h of incubation at 37°C, the color was developed using a substrate solution containing 0.1 M citrate buffer, pH 4.5, 0.03% H₂O₂ and 0.1% of o-phenylenediamine. The absorbence at 490nm in each well was read using an ELISA reader (EL311, BioTek, VT), and the data was analyzed using log-logit model.

### 2. IgM assay:

The procedure for IgM assay was similar to that for IgG assay, except that the samples were 1/10⁴ diluted and standard IgM ranged from 1 µg/ml to 0.0156 µg/ml were added (100µl/well). The secondary antibody for the assay was HRP-conjugated goat anti-mouse IgM (1:1,000 diluted; heavy chain-specific; Zymed Laboratory, CA).

### 3. IgA assay:

The procedure for IgA assay was similar to that for IgG assay, except that the samples were 1/10⁴ diluted and standard IgA ranged from 1 µg/ml to 0.0156 µg/ml were added (100µl/well). The secondary antibody for the assay was HRP-conjugated goat anti-mouse IgA (1:1000 diluted; Zymed Laboratory, CA).

### ELISA to measure concentration of cytokine

For the quantitative analysis of IFN-γ and IL-10; the spleen cells (1x10⁶ cells/ml) were cultured in a medium containing RPMI 1640 supplemented with 10% fetal bovine serum (FBS), 2 mM L-glutamine, antibiotics and 1 µg/ml concanavalin A (Con A) for 3 days. Con A is one of the polyclonal activators for T lymphocyte. Culture spleen cell supernatants were collected. The IFNγ and IL-10 concentrations were measured using a cytokine ELISA set (Cyto Set ELISA kit) purchased from R&D Systems (MN, USA).

### 1. IL-10 assay:

Cyto Set ELISA was performed as follows: A 96-well microtiter plate (Nunc-Immuno Plate, MaxiSorp, Nunc, Denmark) was precoated with capture antibodies at 4°C overnight. The capture antibody was a rat monoclonal antibody to mouse IL-10. The samples to be tested and standard IL-10 ranged from 500 pg/ml to 15.6 pg/ml were added (100µl/well). The plate was then incubated at 37°C for 20 min. At the end of incubation, the plate was washed five times, followed by adding the biotinylated goat anti IL-10 polyclonal antibody. The color was developed by incubating the plate with a HRP-conjugated streptoavidin (Zymed, CA, USA), followed by a substrate solution containing hydrogen peroxide and tetramethylbenezidine (TMB). The reaction continued for 30 min at room temperature, and was stopped by adding 100 µl of 2N of sulfuric acid. The absorbence at 450nm in each well was read using an ELISA reader (EL311, BioTek, Winooski, VT), and the data was analyzed using log-logit model.

### 2. IFN-γ assay:

Cyto Set ELISA of IFN-γ was similar to that of IL-10, except that the capture antibody was a rat monoclonal antibody to mouse IFN-γ, and secondary antibody was biotinylated goat anti IFN-γ polyclonal antibody.

### Flow cytometric assay to analyze T-cell populations

The *in vivo* effect of PCM on CD3 (pan-T marker), CD4 (helper T cell maker) and CD8 (Cytotoxic T cell marker) molecule expression on T-lymphocytes was evaluated by flow cytometry. CD3, CD4 and CD8 expressions were monitored using a CY-Chrome conjugated hamster anti-mouse CD3 antibody (Becton Dickinson, San Jose, CA), PE-conjugated hamster anti-mouse CD4 antibody (Becton Dikinson, San Jose, CA) and FITC-conjugated hamster anti-mouse CD8 antibody (Becton Dikinson, San Jose, CA), respectively. Briefly, the non-adherent spleen cells were washed three times with sterile cold PBS, followed by adjusting the cells to 1 x 10⁶ cells/ml. Each 1 x 10⁶ cells were mixed with 1 µl of indicated fluorescence-conjugated antibody and incubated at room temperature in the dark for 15 min. After the incubation, the cells were washed with cold PBS and were pelleted by contrifugation at 200x g for 10 min. The pellet was dispersed and mixed with 500 µl of 1.0% of paraformaldehyde. The percentage of CD3⁺ cells in splenic lymphocytes and the percentage of CD4⁺8⁻ and CD4⁻8⁺ cells in CD3⁺ cell population were analyzed in flow cytometry.

### Assay for NK cytotoxicity

For measuring the NK cell-mediated cytotoxicity, a LIVE/DEAD cell-mediated cytotoxicity kit (Molecular Probes, Eugene, OR) was used for the assay. This two-color fluorescence assay allows direct assessment of cell-mediated cytotoxicity and yields cytotoxicity measurements that correlate well with ⁵¹Cr release assay. The target cell using in our assay system was YAC-1 cells (ATCC, TIB-160). Briefly, the exponentially growth YAC-1 cells were harvested and washed with complete culture medium (RPMI 1640 supplemented with 10% fetal bovine serum, 2 mM L-glutamine and antibiotics). The cells were adjusted to 1×10⁶ cells/ml and 20 µl of DiOC₁₈ (3,3'-diocatadecyloxacarbocyanine) were added for each ml of target cells. The cells were incubated at 37°C for 20 min. The DiOC₁₈ reagent created a green fluorescence membrane stain on the target cells. The cells were then washed twice with PBS and suspended in complete culture medium at a concentration of 2×10⁵ cells/ml. Non-adherent spleen cells (effector cells) were washed with Hank's balance salt solution for three times and suspended in complete culture medium at a concentration of 1 x 10⁷ cells/ml. The effector cells were then two-fold diluted to 5 x 10⁶ cells /ml and 2.5 x 10⁶ cells/ml. To initiate the assay, the equal volume (e.g. 200 µl) of effector cells and target cells was mixed to make the final Effector:Target ratio (E:T ratio) to 50:1, 25:1 and 12.5:1, respectively. The cell mixture was incubated at 37°C for two hours, followed by adding 50 µl of propidium iodide. The propidium iodide can penetrate into the cells with damaged plasma membranes and stain the nuclear with red fluorescence. The cells were pelleted by centrifugation at 1000x g for 30 seconds and incubated at room temperature for another 10 min. After the incubation period, the tube was tapped gently to dislodge the pellets and then vortexed to re-suspend completely. The samples were ready for analyzing in the flow cytometry. The dead target cells had coincident green-membrane and red-nuclear staining. The live target cells were green on the membrane. However, the dead effector cells had only red-nucleus staining.

### Assay for phagocytosis

The phagocytic function of peritoneal macrophages was assayed using a Vybrant Phagocytosis Assay Kit purchased from Molecular Probes Co. (Eugene, OR). BALB/c mice were divided into four groups and oral administration with 1 ml of PCM-K1, ranging from 2.5mg /kg/day to 20 mg/kg/day for four consecutive days. The control group was composed of mice injected with an equal volume of saline (0.85% of NaCl). The animals were i. p. injected with 2.5 ml of 10% proteose peptone at day two and sacrificed by cervical dislocation at day five. The peritoneal cavity was injected with 2 ml of the divalent cation-free, serum-free DMEM. The cavity was then gently massaged for 30 second, and the peritoneal fluid together with medium and peritoneal exudate cells were withdrew using a 25G syringe. The cells were washed with complete DMEM culture medium and adjusted to 1 x 10⁷ cells/ml. The cells were incubated on ice for 10min, followed by mixing 1x10⁶ cells with 5 x10⁶ fluorescent labeled particles. The particles for phagocytosis assay were fluorescein-laeled *Escherichia coli* (K-12 strain) BioParticles (Molecular Probes, Eugene, OR). The cells were divided into experimental group and control group. The experimental group was incubated for 15min at 37°C, followed by adding 100µl of ice cold Quenching solution (1.25 mg/ml trypan blue). The control group was incubated at 0°C, instead. The cells from both groups were washed twice with PBS and pelleted by centrifugation at 150x g for 5 min. The contaminated erythrocytes were removed by adding ACK lysing solution. The cells were re-pelleted by centrifugation at 150x g for 5 min, and the pellet was dispersed and mixed with 500 µl of 1.0% of paraformaldehyde. The amount of particles been phagocytized by the peritoneal macrophages was analyzed by flow cytometry.

### Statistics

Data from the control or drug-treatment groups were tested by ANOVA. The difference between the two means was assessed using the Mann-Whitneys rank sum test. Probability values of < 0.01 were considered to be significant.

### RESULTS

### (1) Spleen Cell growth - Effects of PCM and PCM-K1, K-2, K3, K4

As shown in Table 2.1 and 2.2, at day 3 of *in vitro* culture, spleen cells isolated from mice treated with 40 mg/kg/day of PCM, 5. mg/kg/day or higher dose of PCM-K1, -K3, -K4, 10 mg/kg/day or higher dose of PCM-K2, respectively, showed an significantly augmented effect on the cell growth. The result suggested that oral administration of PCM or purified components such as PCM-K1, K2, K3 or K4 in general did affect the growth of immune cells in lymphoid organ.

**TABLE 2.1 Spleen Cell growth - Effects of PCM and PCM-K1, K-2**

| **Components** | **Dosage** | **Incubation time (day)** | | |
|---|---|---|---|---|
| | **(mg/kg/day)** | **3** | **4** | **5** |
| Control | | 0.359±0.076^{a} | 0.481±0.044 | 0.414±0.067 |
| PCM | 10 | 0.453±0.028 | 0.398±0.057 | 0.301±0.059 |
| PCM | 40 | 0.551±0.040* | 0.401±0.031 | 0.445±0.055 |
| PCM | 80 | 0.475±0.083 | 0.410±0.083 | 0.447±0.075 |
| K1 | 2.5 | 0.497±0.080 | 0.533±0.070 | 0.584±0.060* |
| K1 | 5 | 00.642±0.078** | 0.652±0.077 | 0.469±0.054 |
| K1 | 10 | 0.743±0.083** | 0.733±0.035*** | 0.562±0.025* |
| K1 | 20 | 0.634±0.048** | 0.571±0.091 | 0.452±0.049 |
| K2 | 2.5 | 0.533±0.069* | 0.460±0.052 | 0.414±0.054 |
| K2 | 5 | 0.489±0.087 | 0.480±0.072 | 0.527±0.062 |
| K2 | 10 | 0.928±0.078*** | 0.931±0.065*** | 0.585±0.041* |
| K2 | 20 | 0.655±0.075** | 0.605±0.072 | 0.530±0.057 |

| | | | | |
|---|---|---|---|---|
| ^{a} Data are the absorbance at 570 nm from the result of MTT assay; Data are Mean±S.E.M of ten similar experiments : * indicates p<0.05, ** indicates p<0.01, *** indicates p<0.001 from the control group. | | | | |

**TABLE 2.2 Spleen cell growth- Effects of PCM-K3 and K4**

| **Components** | **Dosage** | **Incubation time (day)** | | |
|---|---|---|---|---|
| | **(mg/kg/day)** | **3** | **4** | **5** |
| Control | | 0.373±0.070^{a} | 0.404±0.033 | 0.431±0.044 |
| K3 | 5 | 1.154±0.172*** | 0.841±0.160* | 0.864±0.194 |
| K3 | 10 | 1.020±0.090*** | 0.900±0.193** | 0.957±0.200* |
| K3 | 20 | 1.103±0.081*** | 1.068±0.132*** | 0.943±0.159** |
| K4 | 5 | 0.949±0.101** | 0.981±0.087*** | 0.862±0.085** |
| K4 | 10 | 1.198±0.101*** | 1.273±0.147*** | 1.177±0.078*** |
| K4 | 20 | 1.233±0.040*** | 1.263±0.041*** | 1.061±0.124*** |

| | | | | |
|---|---|---|---|---|
| ^{a} Data are the absorbance at 570 nm from the result of MTT assay; Data are Mean±S.E.M of six similar experiments ; * indicates p<0.05, ** indicates p<0.01, *** indicates p<0.001 from the control group. | | | | |

### (2) Serum levels of immunoglobulins- Effects of PCM and PCM-K1, K-2, K3, K4

Data in Table 3.1 and 3.2 indicated that mice orally treated with 40 mg/kg/day of PCM significantly elevated the serum level of IgG. Mice treated with 5 mg/kg/day or higher dose of PCM-K1, K2 and K4, respectively, also significantly increased the serum level of IgG. By contrast, mice treated with 5 mg/kg/day or higher dose of PCM-K3 showed a significant reduction in the serum level of IgG. Mice orally treated with 40 mg/kg/day of PCM, 10 mg/kg/day of PCM-K1, 2.5 mg/kg/day of PCM-K2, 5 mg/kg/day of PCM-K3 and 5 mg/kg/day of PCM-K4, respectively, showed a significantly increase in the serum level of IgM. Oral administration of 80 mg/kg/day of PCM significantly reduced the level of IgA in mouse serum. However, mice treated with 10 mg/kg/day of PCM-K1 significantly elevated the serum level of IgA. The result from our experimental system showed no effect of PCM- K2, K3 and K4 on the serum concentrations of IgA.

**TABLE 3.1 Serum level of immunoglobulins- Effect of PCM, PCM-K1, PCM-K2**

| **Components** | **Dosage** | **Immunogglobulin concentrations(mg/ml)** | | |
|---|---|---|---|---|
| | **(mg/kg/day)** | **IgG** | **IgM** | **IgA** |
| Control | | 1.81±0.19^{a} | 0.90±0.18 | 3.67±0.54 |
| PCM | 10 | 2.38±0.28 | 1.31±0.13 | 3.40±0.25 |
| PCM | 40 | 4.36±0.88* | 1.96±0.18*** | 2.54±0.28 |
| PCM | 80 | 4.53±0.92* | 2.07±0.21 *** | 2.26±0.26* |
| K1 | 2.5 | 1.57±0.32 | 1.27±0.22 | 3.51±0.33 |
| K1 | 5 | 2.83±0.41 * | 1.38±0.24 | 4.03±0.47 |
| K1 | 10 | 3.00±0.53** | 3.27±0.47*** | 6.57±0.71** |
| K1 | 20 | 3.74±0.62** | 1.46±0.27 | 3.79±0.35 |
| K2 | 2.5 | 1.69±0.24 | 2.82±0.41*** | 2.85±0.36 |
| K2 | 5 | 2.95±0.17** | 3.46±0.61*** | 2.81±0.28 |
| K2 | 10 | 3.75±0.54*** | 2.58±0.38*** | 4.33±0.33 |
| K2 | 20 | 5.11±0.72*** | 5.13±0.95*** | 5.02±0.65 |

| | | | | |
|---|---|---|---|---|
| ^{a} Data are Mean ±S.E.M of ten similar experiments ; * indicates p<0.05, ** indicates p<0.01, *** indicates p<0.001 from the control group. | | | | |

**TABLE 3.2 Serum level of immunoglobulins- Effect of PCM-K3, PCM-K4**

| **Conponents** | **Dosage** | **Immunoglobulin concentrations (mg/ml)** | | |
|---|---|---|---|---|
| | **(mg/kg/day)** | **IgG** | **IgM** | **IgA** |
| Control | | 1.80±0.11 | 1.11±0.13 | 3.15±0.48 |
| K3 | 5 | 1.32±0.09** | 1.63±0.11** | 3.65±0.26 |
| K3 | 10 | 1.28±0.13** | 1.53±0.14* | 3.81±0.46. |
| K3 | 20 | 1.14±0.11** | 1.53±0.13* | 3.55±0.37 |
| K4 | 5 | 3.75±0.18*** | 2.65±0.21** | 3.33±0.13 |
| K4 | 10 | 4.33±0.29*** | 2.86±0.16** | 3.39±0.25 |
| K4 | 20 | 4.18±0.25*** | 2.36±0.16** | 3.54±0.12 |

| | | | | |
|---|---|---|---|---|
| ^{a} Data are Mean ±S.E.M of six similar experiments; * indicates p<0.05, ** indicates p<0.01, *** indicates p<0.001 from the control group. | | | | |

### (3) Immunoglobulin secreted by Splenic B lymphocyte- Effects of PCM and PCM-K1, K-2, K3, K4

### 3.1 IgG secretion by spleen cells in vitro

As shown in Table 4.1 and 4.2, after three days of *in vitro* culture, spleen cells isolated from mice treated with 40 mg/kg/day of PCM, 5 mg/kg/day or higher dose of PCM-K1 and 10 mg/kg/day or higher dose of PCM-K2, respectively, showed a significant increase in IgG secretion. On the contrary, PCM-K3 significantly suppressed the IgG secretion. Spleen cells isolated from the mice treated with 5 mg/kg/day or higher dose of PCM-K4 showed an significantly augmented effect on IgG secretion at day 5. Result from this experiment suggested that oral administration of PCM or purified components such as PCM-K1, K2 or K4 in general did increase the potential of spleen B cells to secret IgG.

**TABLE 4.1 IgG secretion by Spleen Cell - Effects of PCM and PCM-K1, K-2**

| **Components** | **Dosage** | **Incubation time (day)** | | |
|---|---|---|---|---|
| | **(mg/kg/day)** | **3** | **4** | **5** |
| Control | | 7.40±1.07 ^{a} | 10.00±1.42 | 11.21±1.31 |
| PCM | 10 | 4.24±0.69 | 6.00±0.97* | 6.46±0.94* |
| PCM | 40 | 16.37±3.11* | 17.42±3.41 | 13.67±3.73 |
| PCM | 80 | 10.84±3.42 | 8.67±3.33 | 9.20±3.18 |
| K1 | 2.5 | 19.65±5.73 | 19.88±5.26 | 13.34±3.66 |
| K1 | 5 | 41.53±8.68*** | 32.94±8.84*** | 33-27±6.14** |
| K1 | 10 | 38.4±11.09*** | 30.01±7.40*** | 34.4±8.27*** |
| K1 | 20 | 59.5±13.04*** | 65.55±17.30*** | 70.58±20.47*** |
| K2 | 2.5 | 8.32±2.53 | 15.32±3.67 | 9.02±2.34 |
| K2 | 5 | 5.97±2.70* | 10.48±3.77 | 13-34±3.83 |
| K2 | 10 | 12.95±2.17* | 11.94±2.24 | 17.97±2.07** |
| K2 | 20 | 25.93±10.15* | 27.41±10.98* | 22.82±5.96* |

| | | | | |
|---|---|---|---|---|
| ^{a} Data are Mean ±S.E.M of ten similar experiments; * indicates p<0.05, ** indicates p<0.01, *** indicates p<0.001 from the control group. | | | | |

**TABLE 4.2 IgG secretion by Spleen Cell - Effects of PCM-K3, K-4**

| Components | **Dosage** | **Incubation time (day)** | | |
|---|---|---|---|---|
| | (mg/kg/day) | 3 | 4 | 5 |
| Control | | 7.17±0.89 | 12.04±0.46 | 9.55±0.77 |
| K3 | 5 | -3.85±0.73** | 3.43±0.40** | 4.46±0.95** |
| K3 | 10 | 3.18±0.55** | 3.16±0.62** | 3.48±0.43** |
| K3 | 20 | 6.12±0:70 | 7.10±1.39* | 8.56±1.34 |
| K4 | 5 | 12.55±3.08 | 12.99±0.86 | 18.29±0.92** |
| K4 | 10 | 6.54±1.43 | 15.15±5.16 | 22.59±5.35* |
| K4 | 20 | 21.23±3.19** | 19.11±4.23 | 22.56±3.84* |

| | | | | |
|---|---|---|---|---|
| ^{a} Data are Mean ±S.E.M of six similar experiments; * indicates p<0.05, ** indicates p<0.01, *** indicates p<0.001 from the control group. | | | | |

### 3.2 IgM secretion by spleen cells in vitro

Data on Table 5.1 and 5.2 indicated that, after three days of *in vitro* culture, spleen cells isolated from mice treated with 40 mg/kg/day of PCM, 10mg/kg/day or higher dose of PCM-K1, 2.5 mg/kg/day or higher dose of PCM-K2, 10 mg/kg/day of PCM-K3 and 5 mg/kg/day or higher dose of PCM-K4, respectively, showed a significant increase in IgM secretion. Result from this experiment suggested that oral administration of PCM or purified components such as PCM-K1, K2, K3 or K4 did increase the potential of spleen B cells to secret IgM.

**TABLE 5.1 IgM secretion by Spleen Cell - Effects of PCM and PCM-K1, K-2**

| **Components** | **Dosage** | **Incubation time (day)** | | |
|---|---|---|---|---|
| | **(mg/kg/day)** | **3** | **4** | **5** |
| Control | | 138.27±42.55 ^{a} | 171.10±39.90 | 184.29±30.97 |
| PCM | 10 | 184.37±36.35 | 251.78±37.64 | 273.91±33.30 |
| PCM | 40 | 375.07±13.6*** | 352.16±7.6*** | 323.52±13.7*** |
| PCM | 80 | 328.96±24.46*** | 322.79±23.76* | 296.89±12.42** |
| K1 | 2.5 | 181.30±34.26 | 201.03±33.98 | 190.20±38.67 |
| K1 | 5 | 223.32±51.46 | 233.67±31.32 | 249.35±38.32 |
| K1 | 10 | 356.52±28.8*** | 362.17±31.1** | 527.33±41.0*** |
| K1 | 20 | 267.18±58.97 | 280.79±32.08* | 320.44±41.39* |
| K2 | 2.5 | 378.0±55.5*** | 479.8±60.9*** | 254.4±30.8 |
| K2 | 5 | 457.81±87.3** | 507.62±91.9** | 334.75±48.7* |
| K2 | 10 | 540.23±68.8*** | 512.47±4.2*** | 550.54±50.1 *** |
| K2 | 20 | 837.1±114.5*** | 695.2±144.7** | 591.5±108.4*** |

| | | | | |
|---|---|---|---|---|
| ^{a} Data are Mean ±S.E.M of ten similar experiments; * indicates p<0.05, ** indicates p<0.01, *** indicates p<0.001 from the control group. | | | | |

**TABLE 5.2 IgM secretion by Spleen Cell - Effects of PCM-K3, K-4**

| **Components** | **Dosage** | **Incubation time (day)** | | |
|---|---|---|---|---|
| | **(mg/kg/day)** | **3** | **4** | **5** |
| Control | | 121.91±34.35 | 200.75±32.77 | 219.30±18.60 |
| K3 | 5 | 222.42±42.83 | 214.45±28.51 | 277.52±101.63 |
| K3 | 10 | 287.66±44.44* | 294.30±30.68* | 309.51±30.88* |
| K3 | 20 | 372.14±69.48* | 519.53±52.63*** | 606.44±88.10** |
| K4 | 5 | 463.65±76.41*** | 688.17±85.78*** | 649.89±70.09*** |
| K4 | 10 | 514.45±70.30*** | 733.58±95.70*** | 807.32±104.21*** |
| K4 | 20 | 747.6±128.42*** | 746.50±157.76*** | 857.49±92.19*** |

| | | | | |
|---|---|---|---|---|
| ^{a} Data are Mean ±S.E.M of six similar experiments; * indicates p<0.05, ** indicates p<0.01, *** indicates p<0.001 from the control group. | | | | |

### 3.3 IgA secretion by spleen cells in vitro

Results showing on Table 6.1 and 6.2 indicated that, after three days of *in vitro* culture, spleen cells isolated from mice treated with 40 mg/kg/day of PCM, 5 mg/kg/day had a significant reduction in IgA secretion in comparison with control group. However, mice treated with the purified components showed a rather different result. Oral administration of 10 mg/kg/day or higher dose of PCM-K1 resulted in an increase in IgA secretion by spleen cells *in vitro* at day 3 and day 5. Spleen cells isolated from the mice treated with 10 mg/kg/day or higher dose of PCM-K2 and 5 mg/kg/day or higher dose of PCM-K4, respectively, showed a significant increase in IgA secretion. On the contrary, PCM-K3 significantly suppressed the IgA secretion. Result from this experiment suggested that oral administration of PCM had an opposite effect between serum level of IgA and the secretion of IgA by spleen cells. However, the purified components such as PCM-K1, K2 or K4 in general did increase the potential of spleen B cells to secret IgA.

**TABLE 6.1 IgA secretion by Spleen Cell - Effects of PCM and PCM-K1, K-2**

| **Components** | **Dosage** | **Incubation time (day)** | | |
|---|---|---|---|---|
| | **(mg/kg/day)** | **3** | **4** | **5** |
| Control | | 130.48±30.76 ^{a} | 144.72±31.06 | 128.56±35.52 |
| PCM | 10 | 50.67±13.28** | 66.09±8.68* | 74.73±15.17 |
| PCM | 40 | 84.31±8.09 | 94.72±10.64 | 125.41±8.51 |
| PCM | 80 | 36.03±4.10** | 46.16±5.09** | 56.05±7.71* |
| K1 | 2.5 | 118.64±19.79 | 94.65±53.54 | 108.37±16.01 |
| K1 | 5 | 148.60±24.55 | 127.93±66.0 | 148.81±22.97 |
| K1 | 10 | 32.11±48.1*** | 378.5±196.2*** | 422.74±6.67** |
| K1 | 20 | 145.93±26.52 | 144.08±84.36 | 179.0±18.86 |
| K2 | 2.5 | 133.90±33.39 | 181.00±47.98 | 277.13±101.1 |
| K2 | 5 | 155.67±18.66 | 142.89±26.86 | 94.91±12.07 |
| K2 | 10 | 239.64±43.1* | 258.20±46.4* | 260.00±0.93* |
| K2 | 20 | 273.20±54.87* | 211.48±57.82 | 324.7±153.4** |

| | | | | |
|---|---|---|---|---|
| ^{a} Data are Mean ±S.E.M of ten similar experiments; * indicates p<0.05, ** indicates p<0.01, *** indicates p<0.001 from the control group. | | | | |

**TABLE 6.2 IgA secretion by Spleen Cell - Effects of PCM-K3, K-4**

| **Components** | **Dosage** | **Incubation time (day)** | | |
|---|---|---|---|---|
| | **(mg/kg/day)** | **3** | **4** | **5** |
| Control | | 58.21±12.63 | 66.92±17.16 | 73.41±14.64 |
| K3 | 5 | 32.83±7.00 | 27.37±5.83* | 21.96±4.17** |
| K3 | 10 | 26.57±3.68* | 28.97±5.95* | 18.87±2.66** |
| K3 | 20 | 43.08±4.05 | 43.45±5.92 | 30.50±3.60* |
| K4 | 5 | 55.56±4.71 | 79.51±3.74 | 106.69±7.29* |
| K4 | 10 | 66.43±5.26 | 95.39±10.08 | 121.97±12.69* |
| K4 | 20 | 119.59±15.08** | 101.53±25.08 | 133.16±20.09* |

| | | | | |
|---|---|---|---|---|
| ^{a} Data are Mean ±S.E.M of six similar experiments; * indicates p<0.05, ** indicates p<0.01, *** indicates p<0.001 from the control group. | | | | |

### (4) T_{H}1-type and T_{H}2-type Cytokines secreted by splenic T lymphocytes- Effects of PCM and PCM-K1, K-2, K3, K4

T_{H}1-type cytokines, such as interleukin-2 (IL-2), interferon-γ (IFN-γ) induces the cellular immune response. Whereas, T_{H}2-type cytokines, such as interleukin-4 (IL-4), interleukin-5 (IL-5), interleukin-6 (IL-6), stimulates the B lymphocyte-mediated humoral immune response. To demonstrate the role of PCM and its purified components in immunological regulation, the potential of T_{H}1-type and T_{H}2-type cytokines secreted by splenic T lymphocytes was studied. Spleen cells isolated from the mice treated with PCM and its purified components, such as PCM-K1, -K2, -K3 and K4, were cultured for five days in the presence of ConA. The amount of interferon-γ (IFNγ; TH1-type cytokine) and interleukin-10 (IL-10; TH2-type cytokine) secreted by the spleen T lymphocytes was measured (Table 7.1 & Table 7.2). After the mice were fed with 10 mg and 80 mg /kg/day of PCM, 2.5 mg/kg/day or higher dose of PCM-K1, 2.5 mg/kg/day or higher dose of PCM-K2, 5 mg and 10 mg/kg/day of PCM-K3, and 20 mg/kg/day of PCM-K4, respectively, the IFNγ secretion by ConA-stimulated splenic T cells was significantly augmented. However, the spleen cells isolated from the mice treated with 40 mg/kg/day or higher dose had no effect on IL-10 secretion. Mice treated with 2,5 mg/kg/day or higher dose of PCM-K1, 2.5 mg and 20 mg/kg/day of PCM-K2, respectively, significantly increased the secretion of IL-10 by spleen cells. Taken together, PCM was able to positively regulate T_{H}1-type cytokine at relatively low dose but it failed to regulate Th2-type cytokine secretion. However, the purified components, such as PCM-K1 and -K2, were able to augment both T_{H}1-type and T_{H}2-type cytokines secreted.

**TABLE 7.1 IFN-γ and IL-10 secretion by Spleen Cell- Effects of PCM and PCM-K1. K-2**

| **Components** | **Dosage** | **Cytokines (pg/ml)** | |
|---|---|---|---|
| | **(mg/kg/day)** | **IFN-.** | **IL-10** |
| Control | | 254.14±55.68^{a} | 103.91 ±20.36 |
| PCM | 10 | 465.11±26.6** | 115.90±17.29 |
| PCM | 40 | 264.79±48.36 | 77.31±9.31 |
| PCM. | 80 | 433.44±31.10** | 181.97±33.26 |
| K1 | 2.5 | 428.7±38.9* | 170.28±13.0* |
| K1 | 5 | 673.33±96.73** | 178.76±24.56* |
| K1 | 10 | 682.28±73.78** | 176.17±45.24 |
| K1 | 20 | 783.97±76.1*** | 334.7±45.8*** |
| K2 | 2.5 | 414.80±31.3* | 135.71±19.89 |
| K2 | 5 | 432.70±50.22* | 226.48±55.67* |
| K2 | 10 | 348.45±72.56* | 135.62±48.15 |
| K2 | 20 | 457.48±57.60** | 195.27±40.0* |

| | | | |
|---|---|---|---|
| ^{a} Data are Mean ±S.E.M of ten similar experiments; * indicates p<0.05, ** indicates p<0.01, *** indicates p<0.001 from the control group. | | | |

**TABLE 7.1 IFN-γ and IL-10 secretion by Spleen Cell - Effects of PCM-K3. K-4**

| **Components** | **Dosage** | **Cytokines (pg/ml)** | |
|---|---|---|---|
| | **(mg/kg/day)** | **IFN-.** | **IL-10** |
| Control | | 124.25±28.15 ^{a} | 107.80±20.79 |
| K3 | 5 | 917.07±130.41* | 262.09±108.41 |
| K3 | 10 | 449.74±100.67* | 86.48±33.26 |
| K3 | 20 | 176.20±45.96 | 98.74±27.05 |
| K4 | 5 | 240.45±107.83 | 128.91±45.46 |
| K4 | 10 | 252.26±103.76 | 197.39±68.73 |
| K4 | 20 | 292.00±77.77* | 155.91±26.16 |

| | | | |
|---|---|---|---|
| ^{a} Data are Mean ±S.E.M of six similar experiments; * indicates p<0.05, ** indicates p<0.01, *** indicates p<0.001 from the control group. | | | |

### (5) Spenic T lymphocyte population- - Effects of PCM

CD4⁺8⁻ T-cells are predominantly helper T-cells (T_{H}), and CD4⁻8⁺ T-cells are mainly cytotoxic T-lymphocytes (CTL). To demonstrate the role of PCM and its purified components in modulation of T lymphocyte sub-population, the percentages of CD4⁺ and CD8⁺ cells in non-adherent spleen cell were analyzed by FASC. Mice were fed with PCM ranged from 10 mg/kg/day to 80 mg/kg/day for four consecutive days. The animals were sacrificed at day 5 and non-adherent spleen cells isolated. The PCM-treated mice showed no significant effect on the percentage of CD4⁺8⁻ cells (Table 8). although there was a tendency of increase as the dose was raised to 80 mg/kg/day. However, the PCM-treated mice showed a significantly increase in CD4⁻8⁺ cells subset (Table 8). In other words, the cytotoxic T lymphocyte population was increased after the mice were fed with PCM. The differentiation of CTL is mainly induced by Th1-type cytokines. Therefore, this finding correlated well with the augmented effect of PCM on IFN-γ secretion. Since the CD4⁺8⁻ cells subset also showed a tendency of increase, we did not observed an increase in CD4/CD8 ratio in spleen lymphocyte population.

**TABLE 8 Regulation of T-Ivmphocyte subpopulation effect of PCM**

| **Components** | **Dosage** | **Fluorescent-positive cells (%)** | | |
|---|---|---|---|---|
| | **(mg/kg/day)** | **CD4** | **CD8** | **CD4/CD8** |
| Control | | 23.40±2.31 ^{a} | 12.50±0.82 | 1.87 |
| PCM | 10 | 28.67±2.54 | 15.31±0.72* | 1.87 |
| PCM | 40 | 27.72±0.66 | 16.67±1.08** | 1.66 |
| PCM | 80 | 29.95±2.75 | 16.76±1.29** | 1.79 |

| | | | | |
|---|---|---|---|---|
| ^{a} Data are Mean ±S.E.M of five similar experiments; * indicates p<0.05, ** indicates p<0.01 from the control group. | | | | |

### (6) Cytotoxic activity of NK cell- Effects of PCM

NK cells play a pivotal role in innate immunity. NK cells non-specifically kill the tumor cells (transformed cells) and viral infected cells. Non-adherent spleen cells isolated from PCM-treated mice were immediately used for NK-mediated cytotoxicity assay. Result indicated that PCM induced an increase in NK-mediated cytotoxicity at 10 mg/kg/day (Table 9). When the dose of PCM was increased, the NK cell activity returned to the basal level. The activation of NK cells is mainly stimulated by Th1-type cytokines. Therefore, this finding, correlated well with the augmented effect of PCM on IFN-γ secretion.

**TABLE 9. Nature killer cell activitv-Effect of PCM**

| **Components** | **Dosage** | **Target : Effector** | | |
|---|---|---|---|---|
| | **(mg/kg/day)** | **1: 12.5** | **1: 25** | **1 : 50** |
| Control | | 16.57±0.61 ^{a} | 17.04±1.06 | 16.57±0.61 |
| PCM | 10 | 14.24±2.18 | 20.78±1.25 | 23.12±2.86* |
| PCM | 40 | 18.62±2.62 | 18.20±2.02 | 20.99±3.59 |
| PCM | 80 | 15.54±3.49 | 18.27±2.05 | 16.35±0.90 |

| | | | | |
|---|---|---|---|---|
| ^{a} Data are percentage of target cells (YAC-1 cells) which are killed by the NK cells in spleen cell population. The dead cells are propedium iodide-positive; Data are Mean ±S.E.M of five similar experiments; * indicates p<0.05 from the control group. | | | | |

### (7) Phagocytic activity of macrophages- Effects of PCM

Phagocytic cells are involved in the first line defense against invading pathogens. The predominant phagocytic cells in innate immunity are neutrophils and macrophages. In this study, the peritoneal exudated macrophages were fed with the fluorescent-labeled *E*. *coli,* and assayed for the phagocytic activity of elicited macrophages. An average 20.88% of macrophages isolated from control group showed phagocytic activity. However, the percentage of cells showing phagocytic activity was 27.49% and 38.22% after the mice were fed with PCM at 40 mg/kg/day and 80 mg/kg/day, respectively, suggesting that PCM significantly enhance the phagocytic activity of peritoneal macrophages.

**TABLE 10. Phagocytosis of peritoneal exudates macrophages-Effect of PCM**

| **Components** | **Dosage** | **Phagocytosis** |
|---|---|---|
| | (mg/kg/day) | |
| **Control** | | 20.88±3.90 ^{a} |
| PCM | 10 | 17.08±1.82 |
| PCM | 40 | 27.49±3.99* |
| PCM | 80 | 38.22±2.20 ** |

| | | |
|---|---|---|
| ^{a} Data are percentage of macrophages that ingests fluorescent-labeled *E. coli;* Data are Mean ±S.E.M of ten similar experiments; * indicates p<0.05, ** indicates p<0.01 from the control group. | | |

## Claims

1. A *Poria* extract capable of enhancing immunity of a mammal comprising 5-60% of a lanostane having the following chemical formula (I) by weight of the extract, and being substantially devoid of secolanostane: 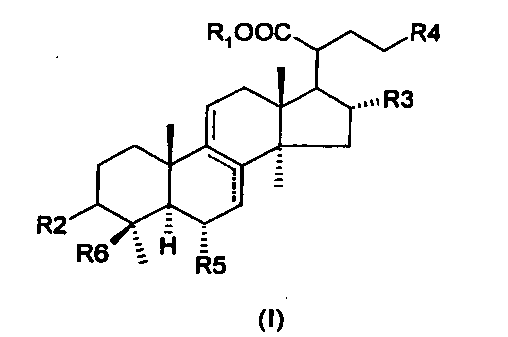 wherein R₁ is either H or CH₃; R₂ is OCOCH₃, =O or OH; R₃ is H or OH; R₄ is -C(=CH₂)-C(CH₃)₂Rₐ, wherein Rₐ is H or OH, or -CH=C(CH₃)-R_{b}, wherein R_{b} is CH₃ or CH₂OH; R₅ is H or OH; and R₆ is CH₃ or CH₂OH.

2. The *Poria* extract according to claim 1, which is prepared by a method comprising the following steps:
a) extracting metabolites, fermentation products or sclerotium of *Poria cocos* (Schw) Wolf by water, methanol, ethanol, or a mixed solvent thereof;
b) concentrating the resulting liquid extract from step a);
c) introducing the resulting concentrated substance from step b) into a silica gel column;
d) eluting the silica gel column with an eluent having a low polarity, and collecting the resulting eluate;
e) concentrating the eluate to form a concentrated eluate.

3. The *Poria* extract according to claim 2, wherein the concentrated eluate from step e) has a chromatographic value, Rf, not less than 0.1 in accordance with a thin layer chromatography, which is developed by a mixed solvent of dichloromethane : methanol = 96:4 and is detected by an ultraviolet lamp and iodine vapor.

4. The *Poria* extract according to claim 2, wherein the extraction in step a) is carried out by using 95% ethanol.

5. The *Poria* extract according to claim 2, wherein the concentrated substance resulted from step b) is further extracted with a two-phase solvent containing methanol and n-hexane in a volumetric ratio of 1:1, a methanol layer is separated from the two-phase solvent extraction mixture, and the methanol layer is concentrated to form a concentrate, which is used as a feed to the silica gel column in step c).

6. The *Poria* extract according to claim 2, wherein the low polarity eluent is a mixed solvent containing dichloromethane and methanol in a volumetric ratio of 96.5:3.5.

7. The *Poria* extract according to claim 1 comprising 10-20% of the lanostane (I).

8. The *Poria* extract according to claim 1, wherein the lanostane (I) is

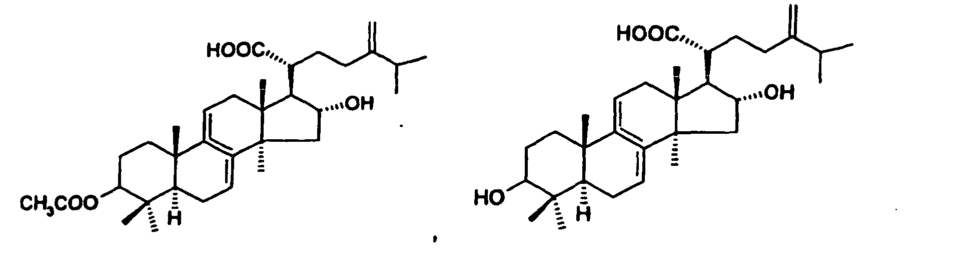

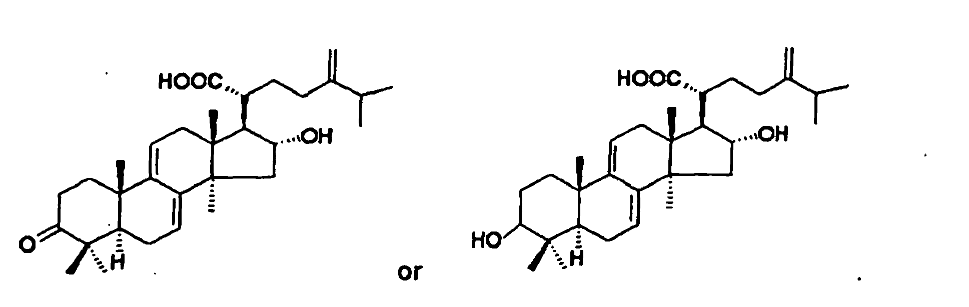

9. Use of the *Poria* extract as claimed in any one of claims 1 to 8 in the manufacture of a medicament for enhancing immunity of mammal.

## Patentansprüche

1. *Poria*-Extrakt, der zur Immunsteigerung eines Säugers befähigt ist, enthaltend 5 bis 60 %, bezogen auf das Gewicht des Extrakts, eines Lanostans mit der folgenden chemischen Formel (I) und der im Wesentlichen frei von Secolanostan ist: 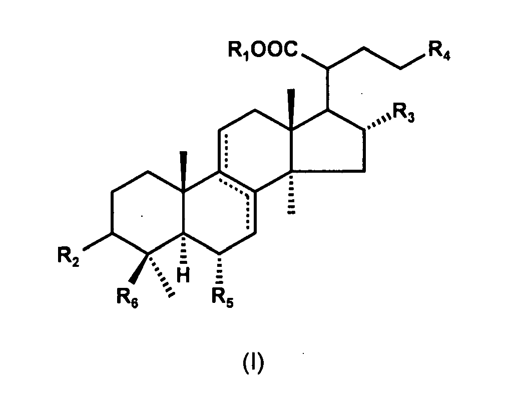 worin R₁ entweder H oder CH₃ ist, R₂ OCOCH₃, =O oder OH ist, R₃ H oder OH ist, R₄ -C(=CH₂)-C(CH₃)₂Rₐ ist, worin Rₐ H oder OH oder -CH=C(CH₃)-R_{b} ist, wobei R_{b} CH₃ oder CH₂OH ist, R₅ H oder OH ist und R₆ CH₃ oder CH₂OH ist.

2. *Poria*-Extrakt gemäß Anspruch 1, der durch ein Verfahren hergestellt ist, das die folgenden Schritte umfasst:
a) Extrahieren von Metaboliten, Fermentationsprodukten oder Sklerotium von *Poria cocos* (Schw) Wolf mit Wasser, Methanol, Ethanol oder einem gemischten Lösemittel davon,
b) Konzentrieren des erhaltenen Flüssigkeitsextrakts aus Schritt a),
c) Einführen der erhaltenen konzentrierten Substanz aus Schritt b) in eine Silicagelsäule,
d) Eluieren der Silicagelsäule mit einem Eluiermittel mit einer niedrigen Polarität und Sammeln des erhaltenen Eluats,
e) Konzentrieren des Eluats zur Bildung eines konzentrierten Eluats.

3. *Poria*-Extrakt gemäß Anspruch 2, worin das konzentrierte Eluat aus Schritt e) einen chromatografischen Wert Rf von nicht weniger als 0,1 bei einer Dünnschichtchromatografie hat, die mit einem gemischten Lösemittel aus Dichlormethan : Methanol = 96 : 4 entwickelt wird und durch eine Ultraviolett-Lampe und loddampf nachgewiesen wird.

4. *Poria*-Extrakt gemäß Anspruch 2, worin die Extraktion in Schritt a) unter Verwendung von 95 % Ethanol durchgeführt wird.

5. *Poria*-Extrakt gemäß Anspruch 2, worin die aus Schritt b) erhaltene konzentrierte Substanz weiter mit einem Zweiphasen-Lösemittel extrahiert wird, das Methanol und n-Hexan in einem volumetrischen Verhältnis von 1 : 1 enthält, eine Methanolschicht aus der Zweiphasen-Lösemittel-Extraktionsmischung abgetrennt wird, und die Methanolschicht zur Bildung eines Konzentrats konzentriert wird, das als Beschickung für die Silicagelsäule in Schritt c) verwendet wird.

6. *Poria*-Extrakt gemäß Anspruch 2, worin das Eluiermittel mit niedriger Polarität ein gemischtes Lösemittel ist, das Dichlormethan und Methanol in einem volumetrischen Verhältnis von 96,5 : 3,5 enthält.

7. *Poria*-Extrakt gemäß Anspruch 1, das 10 bis 20 % des Lanostans (I) enthält.

8. *Poria*-Extrakt gemäß Anspruch 1, worin das Lanostan (I) 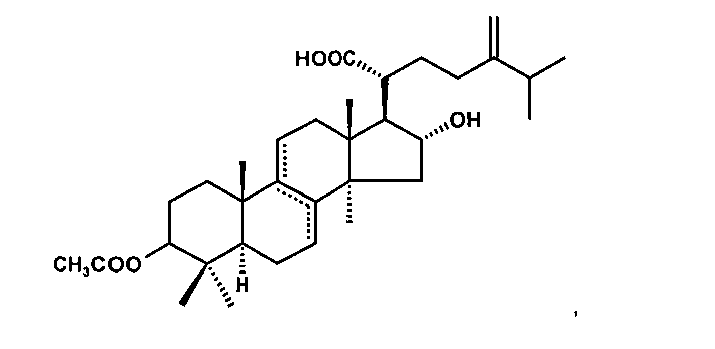 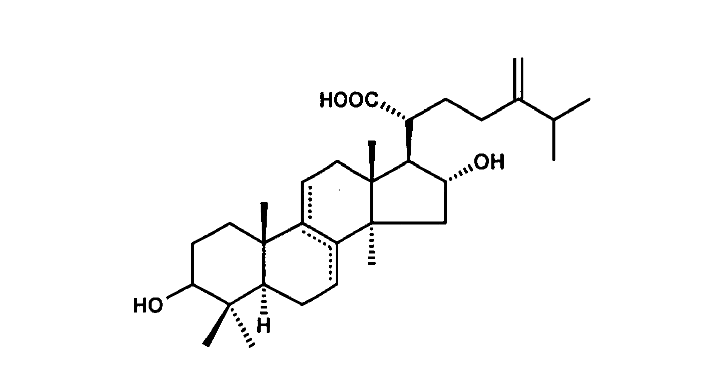 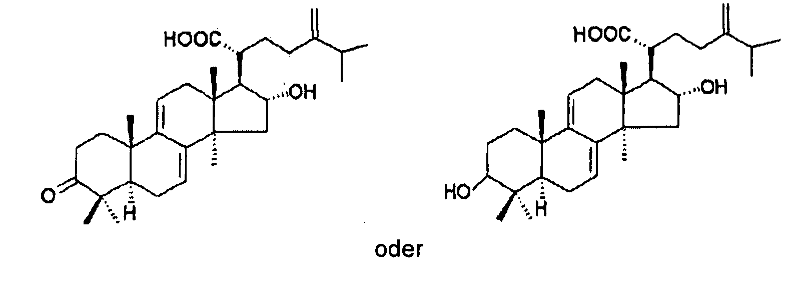 ist.

9. Verwendung des *Poria*-Extrakts wie in einem der Ansprüche 1 bis 8 beansprucht zur Herstellung eines Medikaments zur Immunitätssteigerung eines Säugers.

## Revendications

1. Extrait de *Poria,* capable d'augmenter l'immunité d'un mammifère; comprenant 5-60% en poids de l'extrait, d'un lanostane de formule chimique (I) suivante et pratiquement dépourvu de sécolanostane : 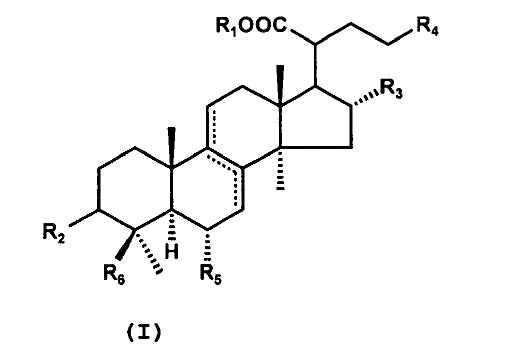 dans laquelle R₁ est soit H soit CH₃ ; R₂ est OCOCH₃, =0 ou OH ; R₃ est H ou OH ; R₄ est -C(=CH₂)-C(CH₃)₂Rₐ, où Rₐ est H ou OH, ou -CH=C(CH₃)-R_{b}, où R_{b} est CH₃ ou CH₂OH ; Rₛ est H ou OH ; et R₆ est CH₃ ou CH₂OH.

2. Extrait de *Poria,* selon la revendication 1, qui est préparé par un procédé comprenant les étapes suivantes :
a) extraction de métabolites, de produits de fermentation ou de sclérotes de *Poria cocos* (Schw)Wolf par de l'eau, du méthanol, de l'ethanol, ou un solvant mixte de ceux-ci ;
b) concentration de l'extrait liquide résultant de l'étape a) ;
c) introduction de la substance concentrée résultante de l'étape b) sur une colonne de gel de silice ;
d) élution de la colonne de gel de silice avec un eluant à faible polarité, et récupération de l'éluat résultant ;
e) concentration de l'éluat pour former un éluat concentré.

3. Extrait de *Poria* selon la revendication 2, dans lequel l'éluat concentré de l'étape e) a un indice chromatographique, Rf, pas inférieur à 0,1 selon une chromatographie en couche mince, est développé par un solvant mixte de dichlorométhane : méthanol = 96 : 4 et est détecté par une lampe ultraviolette et de la vapeur d'iode.

4. Extrait de *Poria* selon la revendication 2, dans lequel l'extraction de 1'étape a) est effectuée en utilisant de l'éthanol à 95%.

5. Extrait de *Poria* selon la revendication 2, dans lequel la substance concentrée obtenue à l'étape b) est encore extraite avec un solvant à deux phases contenant du méthanol et du n-hexane dans un rapport volumétrique de 1 : 1, la couche de méthanol est séparée du mélange d'extraction par le solvant à deux phases, et la couche de méthanol est concentrée pour former un concentré, qui est utilisé comme charge pour la colonne de gel de silice dans l'étape c).

6. Extrait de *Poria* selon la revendication 2, dans lequel l'éluant à faible polarité est un solvant mixte contenant du dichlorométhan et du méthanol dans un rapport volumétrique de 96,5 : 3,5.

7. Extrait de *Poria* selon la revendication 1 comprenant 10-20% du lanostane (I).

8. Extrait de *Poria* selon la revendication 1 dans lequel le lanostane (I) est

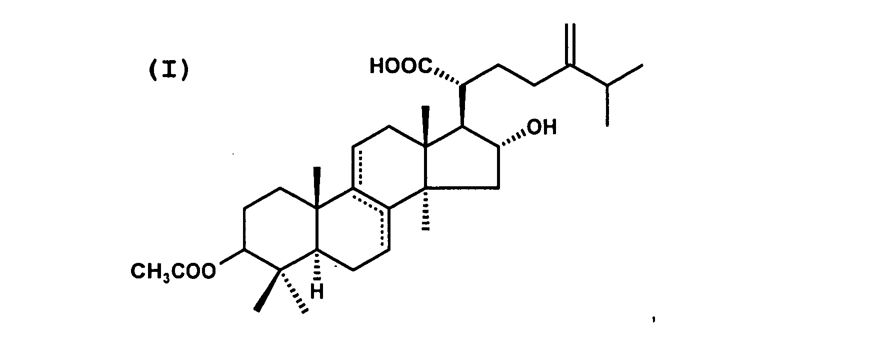

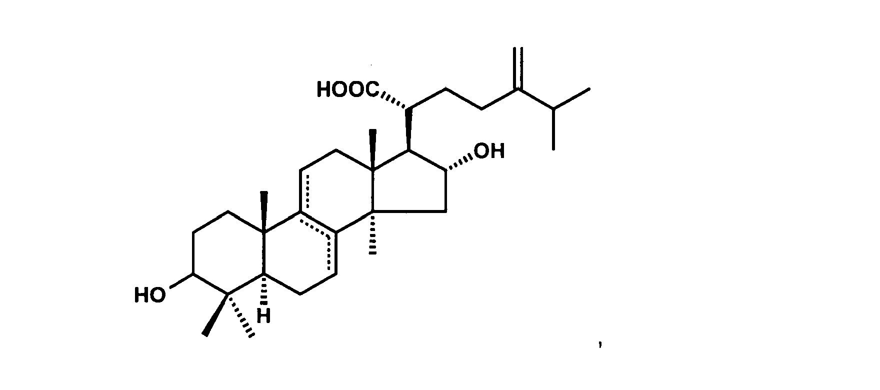

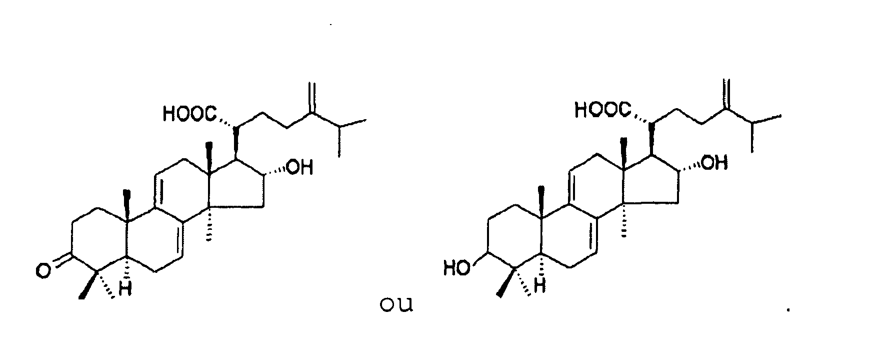

9. Utilisation de l'extrait de *Poria* tel que revendiqué dans l'une quelconque des revendications 1 à 8 dans la fabrication d'un médicament pour augmenter l'immunité d'un mammifère.
